(19) **Europäisches Patentamt / European Patent Office / Office européen des brevets**

(11) **EP 3 684 263 B1**

(12) # EUROPEAN PATENT SPECIFICATION

(45) Date of publication and mention of the grant of the patent:
**15.11.2023 Bulletin 2023/46**

(21) Application number: **18792456.8**

(22) Date of filing: **14.08.2018**

(51) International Patent Classification (IPC):
**A61B 8/08** (2006.01)     **A61N 7/02** (2006.01)
**A61B 8/00** (2006.01)     **A61N 7/00** (2006.01)
**A61B 6/03** (2006.01)     **A61B 34/20** (2016.01)

(52) Cooperative Patent Classification (CPC):
**A61B 8/469; A61B 8/0808; A61B 8/085;**
**A61N 7/00; A61N 7/02;** A61B 6/032; A61B 8/0816;
A61B 8/0858; A61B 8/4227; A61B 8/4494;
A61B 8/481; A61B 8/5207; A61B 8/5246;
A61B 2034/2046; A61B 2034/2055;     (Cont.)

(86) International application number:
**PCT/IB2018/001103**

(87) International publication number:
**WO 2019/058171 (28.03.2019 Gazette 2019/13)**

(54) **FOCAL CAVITATION SIGNAL MEASUREMENT**

FOKALKAVITATIONSSIGNALMESSUNG

MESURE DE SIGNAL DE CAVITATION FOCALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.09.2017 US 201715708214**

(43) Date of publication of application:
**29.07.2020 Bulletin 2020/31**

(73) Proprietor: **Insightec Ltd.**
**39120 Tirat-Carmel (IL)**

(72) Inventors:
• **VITEK, Shuki**
**34467 Haifa, OT (IL)**
• **LEVY, Yoav**
**Hinanit, OT (IL)**

(74) Representative: **Dennemeyer & Associates S.A.**
**Landaubogen 1-3**
**81373 München (DE)**

(56) References cited:
WO-A1-2011/079177     WO-A1-2017/004562
US-A1- 2010 125 192     US-A1- 2010 179 425
US-A1- 2012 157 842

• **RYAN M JONES ET AL: "Paper;Transcranial passive acoustic mapping with hemispherical sparse arrays using CT-based skull-specific aberration corrections: a simulation study;Transcranial passive acoustic mapping with hemispherical sparse arrays using CT-based skull-specific aberration corrections: a simulation study", PHYSICS IN MEDICINE AND BIOLOGY, INSTITUTE OF PHYSICS PUBLISHING, BRISTOL GB, vol. 58, no. 14, 27 June 2013 (2013-06-27), pages 4981-5005, XP020247667, ISSN: 0031-9155, DOI: 10.1088/0031-9155/58/14/4981**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
A61B 2034/2063; A61N 2007/003; A61N 2007/0039

## Description

FIELD OF THE INVENTION

The field of the invention relates generally to ultrasound systems and, more particularly, to systems and methods for measuring a cavitation signal from an ultrasound focus at a target region.

BACKGROUND

Focused ultrasound (i.e., acoustic waves having a frequency greater than about 20 kiloHertz) can be used to image or therapeutically treat internal body tissues within a patient. For example, ultrasound waves may be used in applications involving ablation of tumors, targeted drug delivery, disruption of the blood-brain barrier (BBB), lysing of clots, and other surgical procedures. During tumor ablation, a piezoceramic transducer is placed externally to the patient, but in close proximity to the tumor to be ablated (i.e., the target region). The transducer converts an electronic drive signal into mechanical vibrations, resulting in the emission of acoustic waves. The transducer may be geometrically shaped and positioned along with other such transducers so that the ultrasound energy they emit collectively forms a focused beam at a "focal zone" corresponding to (or within) the target region. Alternatively or additionally, a single transducer may be formed of a plurality of individually driven transducer elements whose phases can each be controlled independently. Such a "phased-array" transducer facilitates steering the focal zone to different locations by adjusting the relative phases among the transducers. As used herein, the term "element" means either an individual transducer in an array or an independently drivable portion of a single transducer. Magnetic resonance imaging (MRI) may be used to visualize the patient and target, and thereby to guide the ultrasound beam.

During a focused ultrasound procedure, small gas bubbles (or "microbubbles") may be generated and/or introduced into the target region. Depending upon the amplitude and frequency of the applied acoustic field, the microbubbles may oscillate or collapse (this mechanism is called "cavitation") and thereby cause various thermal effects in the target region and/or its surrounding region. For example, at a low acoustic pressure, cavitation of microbubbles may enhance energy absorption at the ultrasound focal region such that the tissue therein may be heated faster and be ablated more efficiently than would occur in the absence of microbubbles. If utilized in the central nervous system, microbubble cavitation may cause disruption of blood vessels, thereby inducing "opening" of the BBB for enhancing targeted drug delivery. However, at a high acoustic pressure, unstable microbubble cavitation may be induced; this may cause undesired bio-effects such as hemorrhage, cell death, and extensive tissue damage beyond that targeted.

To minimize the undesired effects of microbubble cavitation during the ultrasound procedure, one conventional approach associates a cavitation detector with the ultrasound transducer to measure cavitation signals (e.g., a pressure wave) from the microbubbles after each ultrasound sonication; if the cavitation signal level is above a predefined threshold amplitude, the ultrasound procedure is suspended. The cavitation signals before being received by the cavitation detector, however, have to traverse one or more layers of intervening tissue (e.g., the patient's skull and scalp) located between the cavitation detector and the target; the cavitation signals may interact with the intervening tissue through multiple processes, including propagation, scattering, absorption, reflection, and refraction. As a result, besides the propagating signals, the cavitation detector may also detect the reflected, refracted, and/or scattered cavitation signals, and can thereby fail to provide information accurately reflecting the cavitation effect on the target region. Although it may be possible to filter the undesired cavitation signals, this would require deployment of numerous cavitation detectors in order to obtain a large enough cavitation signal relative to the noise to be filtered. This may significantly increase the design and economic burden.

WO2017/004562 discloses systems and methods for modulation and mapping of brain tissue using an ultrasound assembly.

Accordingly, there is a need to accurately detect and monitor microbubble cavitation resulting from ultrasound waves at the target region without the burden of employing large numbers of cavitation detectors.

SUMMARY

The present invention provides systems and methods as set out in the claims. These are suitable for accurate and reliable detection of microbubble cavitation in an ultrasound focus at the target region and/or its surrounding region during an ultrasound procedure (such as ultrasound therapy or imaging) without the need for large numbers of cavitation detectors. In various embodiments, a limited number (preferably less than five, or less than 10) cavitation detection devices are brought into direct contact with a patient's scalp at regions providing high transmission efficiency (e.g., above a predetermined threshold) for the cavitation signals. Transmission efficiency associated with each scalp region can be computed based on the predicted beam path from the target region through the skull and scalp, and anatomical characteristics of the scalp and/or skull in the intercepted regions. The anatomical characteristics may be acquired using an imaging system (e.g., an MRI device and/or a computer tomography device). In addition, the locations of the scalp where the cavitation detection devices are to be attached may be selected by taking into account other characteristics (e.g., geometry) of the scalp and/or skull regions. For example, a skull region having a substantially flat surface and a scalp region having no scars may be preferred. Cavitation detectors may be attached to a patient's scalp using, for example, a conductive paste or gel.

Once the locations of the scalp/skull regions for attaching the cavitation detection devices thereto are determined, the coordinates of the scalp/skull regions in the imaging (e.g., MRI) system are registered to the spatial coordinates of the environment where the patient is located. In various embodiments, the registration is performed by attaching at least three locational trackers to at least three MRI fiducials (e.g., the patient's nose tip, ears, eyes edges or upper jaw teeth). An optical image of the locational trackers may be acquired in real time; this allows the user to infer the spatial coordinates of the trackers in the environment based on the real-time image. In various embodiments, based on the locations of the MRI fiducials in an MR image and the locations of the trackers in the optical image, a registration matrix transforming the MRI coordinates to the coordinates of the optical imaging system can be obtained. Thereafter, movement of the trackers in the spatial coordinates can be monitored using the optical images, which can then be transformed into the MRI coordinates using the registration matrix. In some embodiments, the patient's scalp and the locational trackers are displayed (as an MR image or an optical image) to a user for assisting attachment of the cavitation detection devices to the preferred scalp regions (e.g., regions having high transmission efficiency). For example, the preferred scalp regions may be emphasized on the display using highlights or circles and the real-time locations of the trackers may be superimposed on the scalp image. The user can then move the locational trackers until their locations overlap satisfactorily with the emphasized scalp regions, and subsequently attach the cavitation detection devices to the scalp locations indicated by the locational trackers. Alternatively, the scalp may be displayed using various colors, each corresponding to the transmission efficiency of the cavitation signals when traversing the corresponding skull region. Again, the user may utilize the locational trackers to guide attachment of the cavitation detection devices to the scalp regions having high transmission efficiency.

The cavitation detection devices may be wired or wireless devices and may detect signals in the time domain and/or frequency domain. In some embodiments, the cavitation detection devices are off-the-shelf products (e.g., conventionally available transceivers) or modified using the off-the-shelf products.

In various embodiments, the signal-to-noise ratio (SNR) of the received cavitation signals is improved by optimizing the configuration and/or properties of the housing accommodating the cavitation detection devices. For example, the geometry of the housing may be tailored to be complementary to the geometry of the skull to avoid gaps between the transducer and the patient's head. This can be achieved by, for example, including a gel or other suitable conforming (but acoustically minimally-interfering) material along the surface of the housing or manufacturing the housing based on the geometry of the patient's skull using, for example, a camera scan or a computed tomography (CT) scan of the patient's head to guide a three-dimensional printer.

Alternatively or in addition, the cavitation detection device may be oriented in alignment with the propagating direction of the acoustic signals so as to reduce reflections occurring at the surface of the cavitation detection device. In some embodiments, the acoustic delay of the cavitation signals is optimized by adjusting the propagation distance of the acoustic signals traversing the skull and housing prior to reaching the cavitation detection device. In addition, the material properties of the housing may be selected or adjusted to provide impedance matching between the skull and the cavitation detection device to assure that maximum signal power is received by the cavitation detection device.

Alternatively or additionally, the housing may include an acoustic impedance-matching layer to provide impedance matching between the skull and the cavitation detection device. Because different patients may have different skull impedances, tailoring the material properties of the housing and/or employing an impedance-matching layer may significantly improve performance. In some embodiments, the housing further includes one or more acoustic absorbers and/or reflectors to absorb/reflect signals originating from sources other than the target region. The absorbers/reflectors may also allow the cavitation detection device to receive fewer signals that have been refracted, reflected and/or scattered and which therefore cannot provide information accurately reflecting cavitation within the target region. In addition, the cavitation detection device(s) may be arranged with respect to the target region such that the SNR of the received cavitation signals is larger than $10^{-6}$ (or in some embodiments, larger than one).

Accordingly, in one aspect, the invention pertains to a system for detecting cavitation signals from a target region of a patient during a focused ultrasound procedure. The system includes an ultrasound transducer; an imaging device for acquiring physiological characteristics of a plurality of anatomical regions through which the cavitation signals from the target region travel; a controller; and at least one cavitation detection device attached to the corresponding skin region. The controller is configured to select at least one of the anatomical regions based at least in part on the physiological characteristics thereof and map the selected anatomical region to a corresponding skin region. In some embodiments, the system further includes display hardware for displaying the corresponding skin region. In addition, the controller may be further configured to operate the ultrasound transducer based at least in part on the cavitation signals received by the cavitation detection device(s).

In various embodiments, the controller is further configured to predict a beam path and beam aberrations of a cavitation signal travelling through each of the anatomical regions from the target region based on the physiological characteristics of the anatomical regions along the beam path. In addition, the controller may be configured to predict transmission efficiency associated with each of the anatomical regions based on the physiological characteristics along the beam path. The physiological characteristics may include structure, thickness, the number of layers, the local bone density, surface geometry, and/or the incidence angle of the beam path associated with each of the anatomical regions. In one embodiment, the controller is configured to select the anatomical region(s) based on the transmission efficiency associated therewith. The controller may be further configured to map the selected anatomical region(s) to the corresponding skin regions by projecting the predicted signal path from the target region onto the corresponding skin regions.

In various embodiments, the controller is configured to correlate coordinates of the imaging device with spatial coordinates in a room in which the patient is located. In addition, the system may include a secondary imaging device for acquiring a real-time image of three or more locational trackers and/or acquiring the physiological characteristics of the target region and/or corresponding skin region. The locational trackers may be attached to three fiducials, and the locational trackers and/or the fiducials may be detectable by the imaging device. The controller may be configured to register coordinates in the secondary imaging device to coordinates in the imaging device. In one embodiment, the controller is configured to register coordinates in the secondary imaging device to coordinates in the imaging device.

Another aspect of the invention relates to a method of placing at least one cavitation detection device for detecting cavitation signals from a target region of a patient during a focused ultrasound procedure. The method includes (a) acquiring characteristics of a plurality of anatomical regions through which the cavitation signals from the target region travel; (b) selecting at least one of the anatomical regions based at least in part on the characteristics thereof; (c) mapping the selected anatomical region to a corresponding skin region; and (d) based on the mapping, placing the at least one cavitation detection device on the corresponding skin region.

There is also provided a system for detecting cavitation signals from a target region of a patient during a focused ultrasound procedure. The system includes an ultrasound transducer; a housing configured for engagement with an anatomical region through which the cavitation signals from the target region travel; and a cavitation detection device inside the housing for detecting the cavitation signals from the target region. In one implementation, at least a portion of the housing is optimized for cavitation detection. For example, the housing may be optimized by configuring the surface geometry thereof to be complementary to the surface geometry of the anatomical region.

In addition, the orientation of the cavitation detection device may be aligned with the propagating direction of the cavitation signals. The housing may be configured to provide a delay length for the cavitation signals to travel therethrough. The delay length may be represented as d2 and may satisfy the equation:

$$d_2 = n \times \frac{\lambda}{2} - d_1,$$

where $d_1$ represents the delay length of the anatomical region through which the cavitation signals travel; $\lambda$ represents the wavelength of the cavitation signals; and $n$ is an integer.

The system may include an acoustic impedance-matching layer inside the housing for matching acoustic impedances of the anatomical region and the cavitation detection device. In addition, the system may further include an acoustic absorber inside the housing for absorbing noise other than the cavitation signals. Additionally or alternatively, the system may include an acoustic reflector (e.g., an air gap) inside the housing for reflecting noise other than the cavitation signals. In one implementation, the housing is configured to provide a propagation width for the cavitation signals to travel therethrough. The propagation width is represented as Dh and satisfies the equation:

$$\left(\frac{v_s}{D_s} + \frac{v_h}{D_h}\right) \times 2 = nT,$$

where $D_s$ represents the width of the anatomical region through which the cavitation signals travel; $v_s$ represents the acoustic velocity in the anatomical region; $v_h$ represents the acoustic velocity in the housing; $T$ represents the period of the cavitation signals; and $n$ is an integer. In some embodiments, the housing is configured to increase the signal-to-noise ratio of the detected cavitation signals.

There is also provided a system for detecting cavitation signals from a target region of a patient during a focused ultrasound procedure. The system includes an ultrasound transducer and one or more cavitation detection devices for detecting the cavitation signals from the target region. In one implementation, the cavitation detection device is arranged with respect to the target region such that a SNR of the detected cavitation signals is larger than $10^{-6}$ (or in some embodiments, larger than one).

As used herein, the term "substantially" means ±10%, and in some embodiments, ±5%. Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

BRIEF DESCRIPTION OF THE DRAWINGS

In the drawings, like reference characters generally refer to the same parts throughout the different views. Also, the drawings are not necessarily to scale, with an emphasis instead generally being placed upon illustrating the principles of the invention. In the following description, various embodiments of the present invention are described with reference to the following drawings, in which:

FIG. 1 illustrates a focused ultrasound system;
FIG. 2A schematically depicts microbubbles generated and/or injected in a target region;
FIG. 2B schematically depicts acoustic signals emanating from microbubble cavitation;
FIG. 3A schematically illustrates tissue layers of a human head;
FIG. 3B depicts multiple skull regions, each associated with a cavitation signal path from the target region;
FIGS. 4A and 4B illustrate approaches for mapping a selected skull region to a scalp region;
FIG. 5 depicts registration of coordinates in an imaging system with the spatial coordinates of the environment where the patient is located;
FIG. 6 illustrates scalp regions that are color- or shade-mapped or otherwise emphasized;
FIG. 7 is a flow chart illustrating an approach for detecting microbubble cavitation signals from the target region using cavitation detection devices directly attached to selected patient's scalp regions;
FIG. 8A illustrates an approach for configuring the geometry of a housing hosting the cavitation detection device in order to increase the SNR of the cavitation signals;
FIGS. 8B and 8C illustrate various approaches for adjusting the orientation and location of the cavitation detection device to improve the quality of the received signals; and
FIGS. 8D and 8E depict implementations of an acoustic impedance-matching layer and an acoustic absorber/reflector, respectively, in the housing for improving quality of the received signals.

DETAILED DESCRIPTION

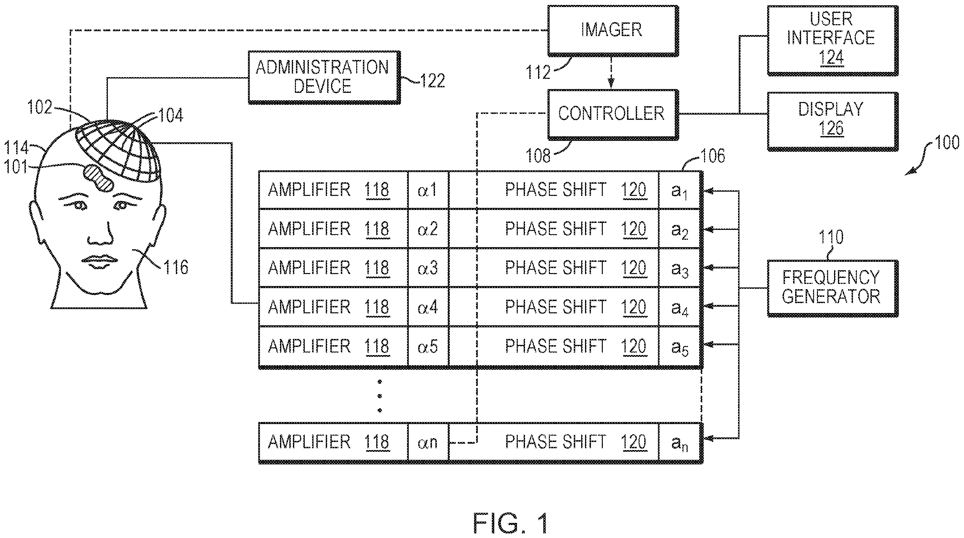

FIG. 1 illustrates an exemplary ultrasound system 100 for generating and delivering a focused acoustic energy beam to a target region 101 within a patient's brain through the skull. One of ordinary skill in the art, however, will understand that the ultrasound system 100 described herein may be applied to any part of the human body. The system 100 includes a phased array 102 of transducer elements 104, a beamformer 106 driving the phased array 102, a controller 108 in communication with the beamformer 106, and a frequency generator 110 providing an input electronic signal to the beamformer 106. The system further includes one or more imaging systems 112, such as an MRI device, a computer tomography (CT) device, a positron emission tomography (PET) device, a single-photon-emission computed tomography (SPECT) device, an optical camera or an ultrasonography device, for acquiring information of the target region 101 and its surrounding region and/or determining anatomical characteristics of the skull 114 of a patient's head 116. The ultrasound system 100 and/or imaging system 112 may be utilized to detect information associated with microbubble cavitation as further described below.

The array 102 may have a curved (e.g., spherical or parabolic) shape suitable for placement on the surface of the skull 114 or a body part other than the skull, or may include one or more planar or otherwise shaped sections. Its dimensions may vary, depending on the application, between millimeters and tens of centimeters. The transducer elements 104 of the array 102 may be piezoelectric ceramic elements or silicon-based elements, and may be mounted in any material suitable for damping the mechanical coupling between the elements 104. Piezo-composite materials, or generally any materials (e.g., silicon devices) capable of converting electrical energy to acoustic energy, may also be used. To assure maximum power transfer to the transducer elements 104 and minimal reflections, the elements 104 may be configured for a specific (i.e., matching) electrical impedance (e.g., 50 $\Omega$).

The transducer array 102 is coupled to the beamformer 106, which drives the individual transducer elements 104 so that they collectively produce a focused ultrasonic beam or field at the target region 101. For n transducer elements, the beamformer 106 may contain n driver circuits, each including or consisting of an amplifier 118 and a phase delay circuit 120; drive circuit drives one of the transducer elements 104. The beamformer 106 receives a radio frequency (RF) input signal, typically in the range from 0.1 MHz to 10 MHz, from the frequency generator 110, which may, for example, be a Model DS345 generator available from Stanford Research Systems. The input signal may be split into n channels for the n amplifiers 118 and delay circuits 120 of the beamformer 106. The frequency generator 110 is integrated with the beamformer 106. The radio frequency generator 110 and the beamformer 106 are configured to drive the individual transducer elements 104 of the transducer array 102 at the same frequency, but at different phases and/or different amplitudes.

The amplification or attenuation factors $\alpha_1$-$\alpha_n$

and the phase shifts $a_1$-$a_n$ imposed by the beamformer 106 serve to transmit and focus ultrasonic energy through the patient's skull 114 onto the target region 101, and account for wave distortions induced in the skull 114 and soft brain tissue. The amplification factors and phase shifts are computed using the controller 108, which may provide the computational functions through software, hardware, firmware, hardwiring, or any combination thereof. For example, the controller 108 may utilize a general-purpose or special-purpose digital data processor programmed with software in a conventional manner, and without undue experimentation, in order to determine the phase shifts and amplification factors necessary to obtain a desired focus or any other desired spatial field patterns. The computation is based on detailed information about the characteristics (e.g., structure, thickness, density, etc.) of the skull 114 and their effects on propagation of acoustic energy. Such information may be obtained from the imaging system 112 as further described below. Image acquisition may be three-dimensional or, alternatively, the imaging system 112 may provide a set of two-dimensional images suitable for reconstructing a three-dimensional image of the skull 114 from which thicknesses and densities can be inferred. Image-manipulation functionality may be implemented in the imaging system 112, in the controller 108, or in a separate device.

An administration device 122 is employed to inject microbubbles the patient's bloodstream, and may either be injected systemically into the patient's brain or locally into the target region 104. The microbubbles may be introduced in the form of liquid droplets that subsequently vaporize, as gas-filled bubbles, or entrained with another suitable substance, such as a conventional ultrasound contrast agent. The administration device 122 may be any suitable apparatus for delivering a suspension of microbubbles into the patient's bloodstream, and can take the form of, e.g., a manual or automated syringe, an intravenous administration bag and needle set, a peristaltic pump, etc. The system 100 further includes a user interface component 124 (including, e.g., a screen, a keyboard, and a mouse) for receiving an input from a user and a display 126 for displaying images of the target tissue 101 and/or intervening tissue to the user.

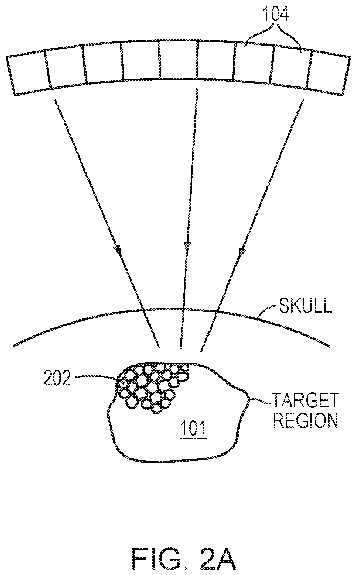

Referring to FIG. 2A, additionally or alternatively, the acoustic energy emitted by the transducer elements 104 may be above a threshold and thereby cause generation of microbubbles 202 in the liquid and/or plasma contained in the target region 101. The microbubbles 202 can be formed due to the negative pressure produced by the propagating ultrasonic waves or pulses, or when the heated liquid ruptures and is filled with gas/vapor, or when a mild acoustic field is applied to tissue that contains cavitation nuclei. The injected and/or generated microbubbles 202 may themselves create or facilitate the creation of additional microbubbles. Therefore, the actual microbubble cavitation effect on the target tissue 101 may result from a combination of the injected and/or directly generated microbubbles and microbubbles that are incidentally created in the tissue.

Generally, at a relatively low acoustic power (e.g., 1-2 Watts above the microbubble-generation threshold), the generated microbubbles 202 undergo oscillation with compression and rarefaction that are equal in magnitude, and thus, the microbubbles 202 generally remain unruptured (a condition known as "stable cavitation" or "streaming cavitation"). The acoustic response of microbubbles 202 is linear at this low acoustic power and the frequency of ultrasound emitted from the microbubbles 202 is the same as, or a harmonic of, that of the incident ultrasound waves (i.e., the fundamental frequency or a base harmonic frequency). At a higher acoustic power (e.g., more than 10 Watts above the microbubble-generation threshold), the generated microbubbles 202 undergo rarefaction that is greater than compression, which may cause cavitation and a nonlinear acoustic response of the microbubbles 202. The acoustic signals returned from cavitation events may include frequencies at the fundamental frequency and/or a harmonic, ultra-harmonic, and/or sub-harmonic of the fundamental frequency. As used herein, the term "fundamental" frequency or "base harmonic" frequency, $f_0$, refers to the frequency (or a temporally varying frequency) of the ultrasound waves/pulses emitted from the transducer array 102; the term "harmonic" refers to an integer multiple of the fundamental frequency (e.g., $2f_0$, $3f_0$, $4f_0$, etc.); the term "ultra-harmonic" refers to a fractional frequency between two nonzero integer harmonics (e.g., $3f_0/2$, $5f_0/4$, etc.); and the term "sub-harmonic" refers to a fractional frequency between the fundamental frequency and the first harmonic (e.g., $f_0/2$, $f_0/3$, $f_0/4$, etc.).

To monitor cavitation effects on the target tissue 101 and/or avoid undesired damage of the target tissue and/or its surrounding tissue resulting therefrom, cavitation events of the microbubbles 202 at the target region 101 are monitored by detecting cavitation signals 204 emanating therefrom using the ultrasound transducer array 102 and/or one or more cavitation detection devices (such as a transceiver or suitable alternative) 206. The cavitation detection devices 206 may be wired or wireless devices in communication with the controller 108, and may detect signals in the time domain and/or frequency domain. The cavitation detection devices 206 may be off-the-shelf products (e.g., conventionally available transceivers). Typically, fewer than five cavitation detection devices 206 are sufficient to provide reliable analysis of the cavitation signals. In some cases, more than five but fewer than 10 cavitation detection devices 206 are necessary.

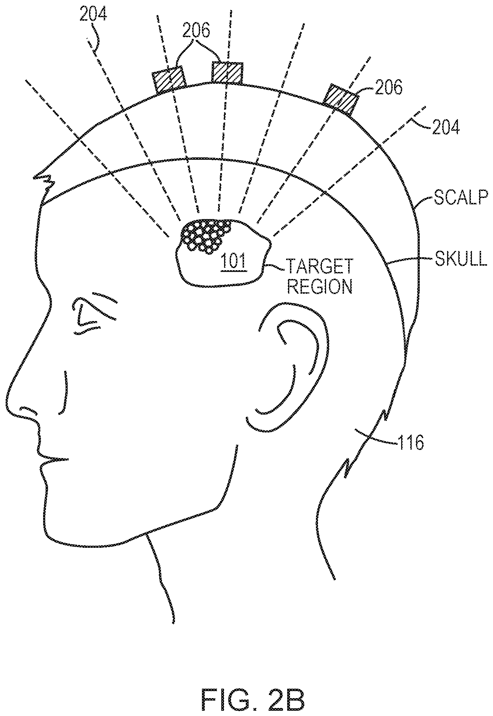

As described above, unlike signals reflected from the microbubbles in which the frequency is the same as that of the incident ultrasound waves, signals emanating from microbubble cavitation include unique spectral signatures (i.e., having a harmonic, ultra-harmonic, and/or sub-harmonic of the incident ultrasound waves). In addition, referring to FIG. 2B, while the directions of the reflection signals highly depend on the locations of the transducer elements 104 and/or amplitude of the exciting acoustic field, the cavitation signals 204 are emitted from a point source (i.e., the location of the microbubble cavitation in the target region 101), and are thereby omnidirectional. The cavitation signals 204 are measured using one or more cavitation detection devices (such as a transceiver or suitable alternative) 206. The detected cavitation signals may be transmitted to the controller 108 for processing and analysis so as to monitor the effect on the target tissue 101 and/or avoid undesired damage of the target tissue 101 and/or its surrounding tissue resulting from the microbubble cavitation. Alternatively, the transducer elements 104 may possess both transmission and detection capabilities. Approaches to detecting cavitation signals of the microbubbles are provided, for example, in U.S. Patent Application No. 15/415,351.

Because the cavitation signals are omnidirectional, the cavitation detection devices 206 may theoretically be placed anywhere on or near the patient's head 116. But because the cavitation signals 204 from the target region 101 must traverse multiple layers of intervening tissue (e.g., the skull and scalp) before reaching the cavitation detection devices 206 and the intervening tissue is typically inhomogeneous, the cavitation signals 204 may be reflected, refracted, absorbed and/or scattered therein. To reduce detection of the reflected, refracted and/or scattered signals and improve quality of the cavitation signals, in various embodiments, the cavitation detection devices 206 are directly attached to the patient's scalp using, for example, a conductive paste or gel, or any other suitable material. In addition, the scalp regions to which the cavitation detection devices 206 are attached may be selected to be located on paths traversed by cavitation signals having sufficiently high transmission efficiency (e.g., above a predetermined threshold, such as 0.5, 0.8 or 0.9, as further described below).

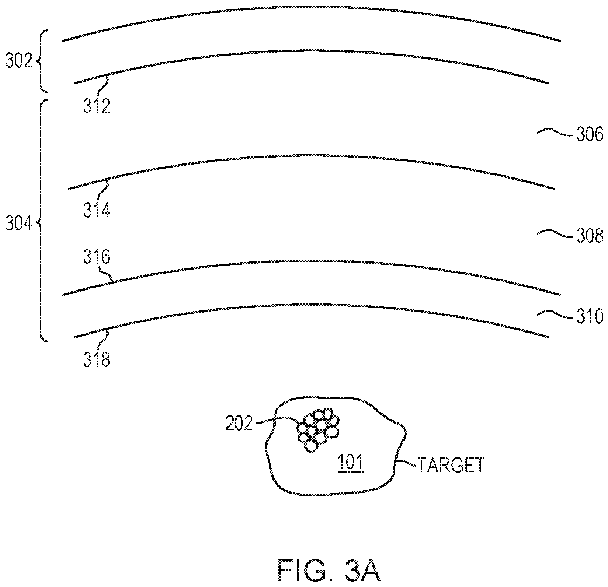

Generally, the cavitation signals 204 propagate evenly in all directions until crossing the intervening skull. Because the anatomical characteristics (such as the structure, thickness, layers, local bone densities and/or directional or geometrical features including a normal relative to the interfaces of the layers) of each skull region may be different, the transmission efficiency associated with various skull regions on various beam paths may vary. Accordingly, in various cases, the transmission efficiency associated with each skull region is determined based on the anatomical characteristics thereof. FIG. 3A schematically illustrates the tissue layers of a human head 116. Typically, the human head includes the scalp 302 and the skull 304, the latter having multiple tissue layers including an external layer 306, a bone marrow layer 308, and an internal layer or cortex 310; each layer of the skull 304 may be highly irregular in shape, thickness and density, and unique to a patient. As a result, when the cavitation signals 204 emitted from the microbubbles 202 at the target region 101 encounter the skull 304, part of the incident acoustic energy may be reflected at the interfaces 312, 314, 316, 318; the remaining energy may be partially absorbed, and partially refracted and propagated through the skull 304 and scalp 302 depending on the frequency of the waves and the structural inhomogeneity of the skull 304 and scalp 302. Because the cavitation signals 204 have unique spectra that can be measured and/or predicted prior to the ultrasound procedure, the effects on signal propagation through various skull regions may be accurately estimated in accordance with the skull features, such as structural inhomogeneity of the skull 304 (e.g., thickness, local density and/or shape of each layer 306-310) and/or incident angles of the cavitation signals entering the skull 304.

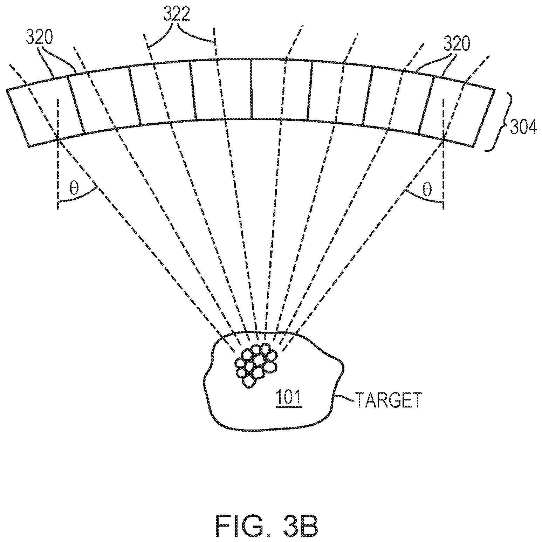

Referring to FIG. 3B, the skull is divided into multiple regions 320, each associated with a cavitation signal path 322 from the target region 101. The skull features associated with each skull region 320 are acquired using images taken by the imaging system 112. For example, a series of images (e.g., CT and/or MR images) of the patient's skull 304 is first acquired prior to the ultrasound procedure. Each image typically corresponds to at least one skull region 320, and the series of images collectively covers the anticipated regions of the skull through which the cavitation signals will travel prior to reaching the cavitation detection devices 206. Alternatively, a three-dimensional image of the skull 304 may be reconstructed using the acquired series of images where the skull regions 320 are generated by the user based on the reconstructed image. In addition, the images may be segmented to define the scalp layer 302 and/or skull layers 306-310. The images of the skull 304 and the target region 101 may be acquired using the same imaging system or different imaging systems. For example, a CT system may be used to acquire the skull features while an MRI system may be used to acquire the features of the scalp and target tissue, as the CT system is well suited for viewing details of bony structures and the MRI system can distinguish subtle changes in soft tissue morphology and function. Coordinates of the two imaging systems can be registered using any suitable registration and/or transformation approach; an exemplary approach is described in U.S. Patent Publication No. 2017/0103533. By applying the imaging registration, images acquired using one system can be transformed into and combined with images acquired using another system.

The images (or combined images) of the skull 304 and the target region 101 are processed to determine the beam paths 322 of the cavitation signals traversing the skull 304 and characterize the skull features associated with the skull regions 320 along the beam paths 322. The characterized skull features may then be utilized to predict aberrations of the cavitation signals through each skull region 320. In one implementation, the skull features are characterized using an indicator that can be quantified at the microstructure level (i.e., having a sensitivity or feature length on the order of a few micrometers, e.g., one, five or 10 micrometers) of the skull 304. For example, the indicator may be a quantified skull density ratio (SDR) created using a skull CT intensity profile obtained from CT images. An exemplary approach for computing the SDR is provided, for example, in U.S. Patent Publication No. 2016/0184026. Upon determining the SDR value associated with each skull region 320, transmission efficiency associated therewith can be determined. For example, the transmission efficiency may have a range between 0 and 1, corresponding to 0% and 100% transmission, respectively, of the cavitation signals through the skull 304. The computed SDR values may have a range with a maximal value; this range may be rescaled into the range of transmission efficiency (i.e., between 0 and 1) using any suitable approach. For example, a linear conversion function may scale the maximal SDR value to the transmission efficiency of 1 and linearly rescale other SDR values into the range of transmission efficiency (i.e., between 0 and 1).

In another implementation, the skull features associated each skull region 320 are characterized using the incident angle, $\theta$, of the cavitation signal entering the skull region. At frequencies of about 2 MHz, the cavitation signals typically propagate with a longitudinal wave mode. Because the velocity of these signals is approximately 2700 m/s in the skull 304, and about 1500 m/s in soft tissue of the brain, signals that arrive at the skull 304 at an incident angle greater than a critical angle (about 30°) are reflected. Accordingly, the transmission efficiency associated with each skull region 320 may be computed based on the incident angle $\theta$ of the cavitation signal entering therein using any suitable function. For example, the transmission efficiency, TE, may be computed as:

$$ TE = e^{\frac{-\theta}{8}}, $$

where $\theta$ has units of degrees.

Further, the transmission efficiency may be computed based on other skull features as well. For example, when the skull region 320 has thickness of approximately ¼ wavelength of the cavitation signals, the cavitation signals may be fully reflected; as a result, the transmission efficiency associated with this skull region 320 may be defined as zero in this region. The transmission efficiency can be defined as a function of more than one parameter (e.g., including both of the SDR and incident angle as variables). The skull regions having transmission efficiencies above a predetermined threshold (e.g., 0.5, 0.8 or 0.9) may then be selected as preferable locations for the cavitation detection devices 206.

Additionally or alternatively, the locations of the cavitation detection devices 206 on the scalp may be selected based on the geometry of the scalp 302 and/or skull 304. For example, because the cavitation detection devices 206 generally have a flat surface, the skull regions having a substantially flat surface are preferred. In addition, it may be desirable to attach the cavitation detection devices 206 to scalp regions that have no scars.

As a result, the locations of the cavitation detection devices 206 are optimized using a cost function including multiple skull features (e.g., the SDR, incident angle, bone thickness, surface geometry, etc.) and/or scalp features (e.g., surface smoothness). For example, the value of the cost function for a skull region having a higher SDR value, a smaller incident angle, a bone thickness substantially thinner than ¼ wavelength of the cavitation signal, and/or a substantially flat surface may be lower than the value of the cost function for a skull region having a lower SDR value, a larger incident angle, a bone thickness substantially equal to ¼ wavelength of the cavitation signal, and/or a curved surface. The cost function employed is not critical and may utilize known or empirically determined cost parameters or constraints. These may be obtained straightforwardly and without undue experimentation based on clinical experience with a small number of patients.

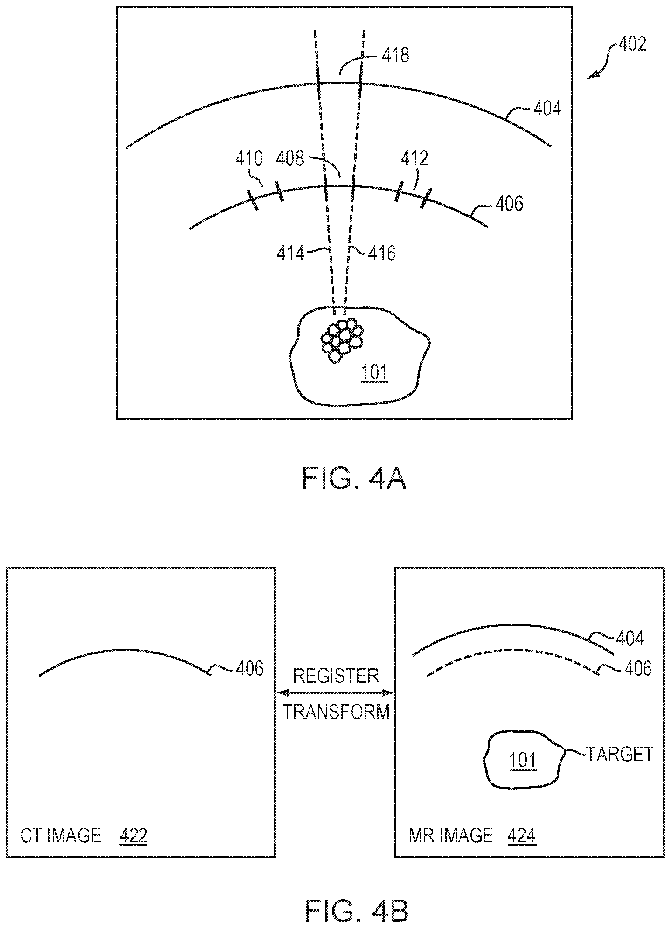

To combine the effects of the skull and scalp features on the cavitation signals and/or attach the cavitation detection devices to scalp regions based on the selected skull regions, it is necessary to map the skull 304 to the scalp 302. Such a map may be obtained using images acquired by one or more imaging systems (such as an MRI imaging system and/or a CT imaging system). For example, referring to FIG. 4A, an MRI image 402 may include the target region 101, the scalp 404 and the skull 406 having multiple defined regions 408-412. To map the skull region 408 to the scalp 404, expected beam paths 414 and 416 connecting microbubbles 202 at the target region 101 to the boundaries of the skull region 408 are computationally projected onto the scalp 404. The projected region 418 is then defined as the scalp region corresponding to the skull region 408. Accordingly, once the skull regions having sufficiently high transmission efficiency and/or a substantially flat surface are selected, their corresponding scalp regions can be mapped/determined, and the cavitation detection devices 206 may then be attached thereto.

Referring to FIG. 4B, the skull features are acquired in a CT image 422 while the scalp and target tissue are acquired in an MR image 424. Again, by registering the two systems, the coordinates in one system can be transformed into coordinates in the other system, and image information in the two systems may be combined in a single coordinate system (preferably the MRI coordinate system). For example, the coordinates of skull regions defined in the CT image 422 may be transformed into MR coordinates; the skull regions may then be displayed on the MR image 424. Once the target region, skull and scalp are all in the same coordinate system, the scalp regions corresponding to the preferred skull regions may be identified using the approach described above in connection with FIG. 4A.

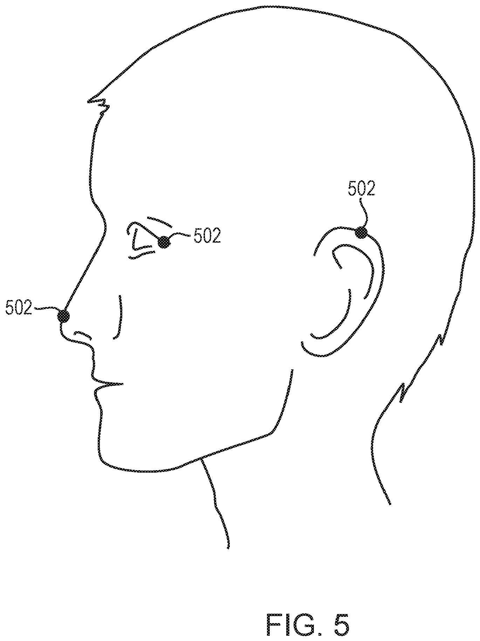

To assist a user with attachment of the cavitation detection devices 206 onto the selected scalp regions, it is necessary to correlate coordinates of the scalp in the imaging system (preferably an MRI system) with the spatial coordinates of the environment where the user is located. Referring to FIG. 5, this is achieved by using at least three locational trackers (e.g., optical trackers and/or RF trackers) 502 and an optical imaging system. For example, the user may first identify three MRI fiducials (e.g., the patient's nose tip, ears, eyes edges, upper jaw teeth, etc.) that are visually detectable and stable and attach three locational trackers 502 thereto. The locations of trackers 502 can be monitored in real time using the optical imaging system; this provides the user with real-time feedback when manipulating the locations of the tracker 502 as described below. In other words, the user may correlate the spatial coordinates of the trackers 502 based on the real-time optical images. Based on the optical images of the trackers 502 and the MR images of the MRI fiducials, a registration matrix transforming the coordinates of the optical imaging system to the MRI coordinates can be computed. Thereafter, the locational trackers 502 may be moved in the environment and tracked in real-time by the optical camera; the new locations of the locational trackers 502 in the MRI coordinates may then be computed using the registration matrix. Alternatively, the locations of the scalp/skull in the MRI coordinates may be transformed into the coordinates of the optical imaging system.

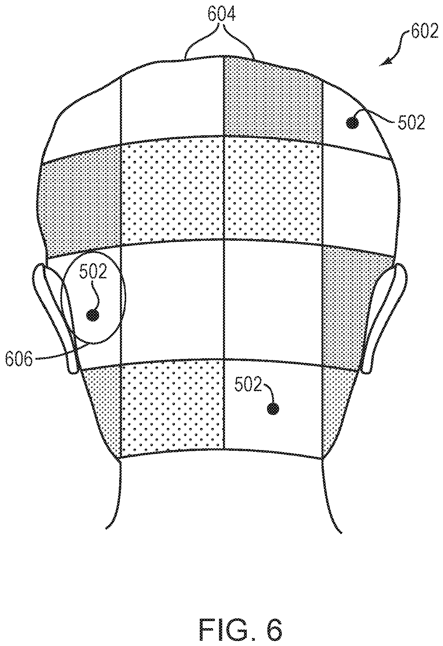

The user is assisted in attaching the cavitation detection devices 206 by an intuitive visual representation of the patient's scalp and the locational trackers 502 in an MR image or an optical image; in some cases, the target locations for the cavitation detection devices 206 are indicated directly on the image, and the user may easily locate the corresponding region on the patient's scalp with reference to the locational trackers 502. Referring to FIG. 6, the patient's scalp 602 may be divided into multiple scalp regions 604 color-mapped by the transmission efficiency of their corresponding skull regions. The locations of the locational trackers 502 may be superimposed on the colored scalp regions 604. The user may then move the locational trackers 502 to the scalp regions having the highest transmission efficiency, and subsequently attach the cavitation detection devices 206 to the locations of the locational trackers 502. Alternatively or additionally, the selected scalp and skull regions to which the cavitation detection devices 206 will be attached may be emphasized in the visual representation (e.g., using highlight or circles 606). Again, the locational trackers 502 may be utilized to guide the user in placing the cavitation detection devices 206.

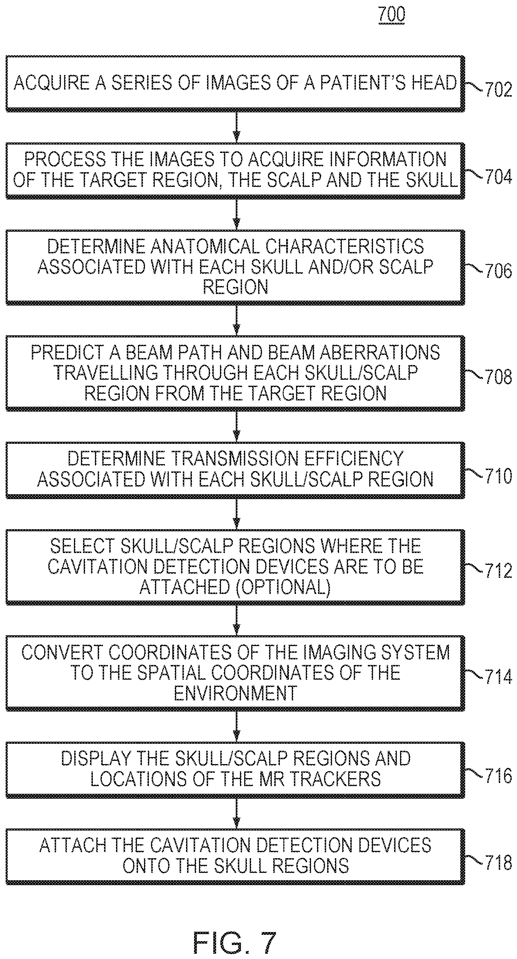

FIG. 7 is a flow chart 700 illustrating an approach for accurately and reliably detecting microbubble cavitation signals from the target region using cavitation detection devices directly attached to selected regions of the patient's scalp that correspond to high transmission efficiency of the cavitation signals. In a first step 702, a series of images of the patient's head is acquired using one or more imaging systems prior to treatment. Each image may include at least one skull region, and the series of images collectively covers the anticipated travel paths of the cavitation signals through the skull. In a second step 704, the images are processed by the controller 108 to identify the locations of the target region 104, the scalp, and the multiple layers of the skull. If the images are acquired using different imaging systems, an image registration may be achieved using any suitable approach and applied to convert the coordinates in one imaging system to the coordinates in another imaging system for analysis. In a third step 706, the images are further analyzed to determine anatomical characteristics associated with each skull region and/or scalp region. In a fourth step 708, based on the anatomical characteristics, the controller may predict a beam path and beam aberrations travelling through each skull/scalp region from the target region. In a fifth step 710, the controller may determine transmission efficiency of the cavitation signals when traversing each skull/scalp region. Optionally, in a sixth step 712, the controller may select skull/scalp regions where the cavitation detection devices are to be attached based on their transmission efficiency and/or other anatomical characteristics (e.g., surface geometry). In a seventh step 714, coordinates of the selected skull/scalp regions in the imaging system may be converted to the spatial coordinates of the environment where the patient is located. This may be performed, for example, using at least three locational trackers attached to three MRI fiducials (e.g., the patient's nose tip, ears, eyes edges or upper jaw teeth, etc.). The locations of the trackers are acquired using an optical imaging system, where the user can correlate the spatial coordinates of the trackers in the environment with the coordinates in the optical imaging system, and the locations of the MRI fiducials are acquired using the MRI system. Based on the optical images of the trackers and the MR images of the MRI fiducials, coordinates in the optical imaging system and MRI system can be registered. In an eighth step 716, the skull/scalp regions selected in step 712 and the real-time locations of the locational trackers may be displayed to the user (as an MR image or optical image). Based thereon, the user can move the locational trackers to the selected regions based on real-time feedback providing by the optical images and then attach the cavitation detection devices thereonto (in a ninth step 718). Alternatively, the skull/scalp regions may be displayed in a color map; each color represents a value of the transmission efficiency. Again, the user may use the locational trackers to guide attachment of the cavitation detection devices to the desired skull/scalp regions (e.g., regions having higher transmission efficiency). During the ultrasound procedure, the cavitation detection devices may then be activated to measure cavitation signals from the target region 101. Based thereon, the cavitation effects on the target tissue and/or its surrounding tissue can be monitored. The ultrasound transducer 102 is operated based on the detected cavitation signals. For example, a parameter (e.g., an amplitude, a frequency, a phase, or a direction) of a transducer element 104 may be adjusted so as to ensure treatment effectiveness while avoiding damage to non-target tissue.

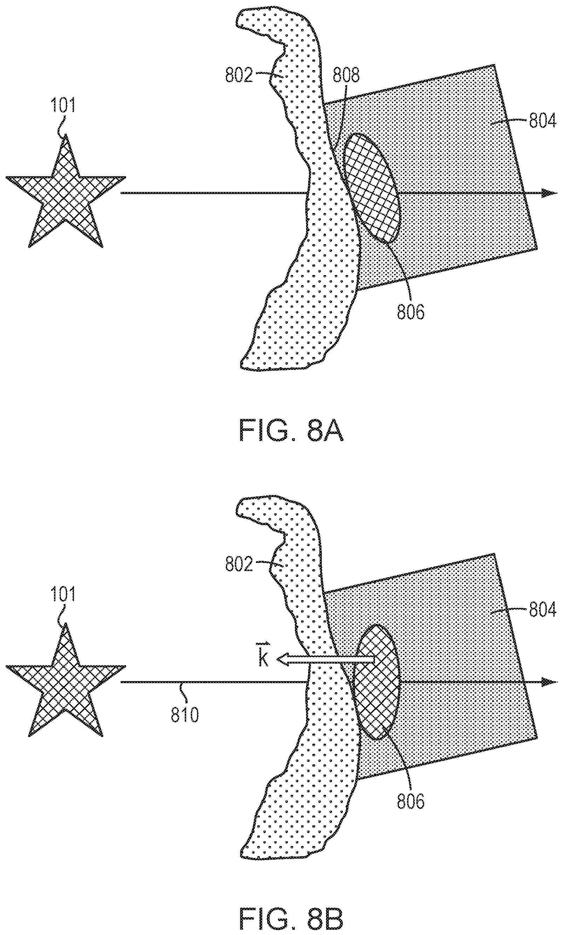

Generally, by bringing the cavitation detection devices 206 into direct contact with the patient's scalp, the SNR of the received cavitation signals 204 is better than that of a cavitation detection device attached to the ultrasound transducer as used in conventional approaches. In addition, by attaching the cavitation detection devices 206 to the scalp regions corresponding to high transmission efficiency, the SNR of the cavitation signals can be further improved. Additionally or alternatively, in various embodiments, the SNR of the received cavitation signals is increased by configuring the geometry of the housing accommodating the cavitation detection devices 206. For example, referring to FIG. 8A, the skull 802 is typically irregular in surface geometry; in various implementations, the housing 804 accommodating the cavitation detection device 806 has a surface geometry complementary to the geometry of the skull 802. In this way, the cavitation detection device 806 can be in intimate overall contact with the skull 802, thereby reducing noise in the received signals. The surface 808 of the housing 806 may be configured by, for example, including a gel or other suitable conforming (but acoustically minimally-interfering) material therealong. Alternatively, the housing 806 may be manufactured using three-dimensional printing techniques based on a camera scan of the patient's skull, with a resulting shape the closely conforms to the surface geometry of the particular patient's skull 802.

In addition, by fully engaging the housing 804 with the skull 802, the orientation and/or location of the cavitation detection device 806 within the housing 804 may be varied to improve performance since there is no longer a need to maximize the contact surface between the cavitation detection device 806 and skull 802 for ensuring engagement therebetween. Accordingly, referring to FIG. 8B, the orientation, k, of the cavitation detection device 808 is aligned with the propagation direction 810 of the acoustic signals from the target region 101; because the intensity of the acoustic field may be highly directionall in the propagation direction 810, this approach may increase the detection efficiency of the receiving device 806.

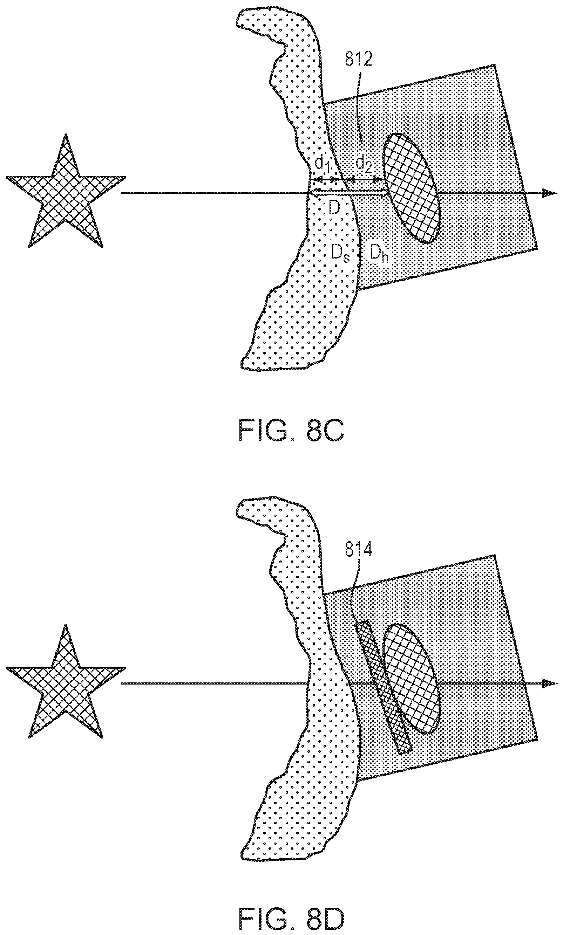

Additionally or alternatively, it may be desired to optimize the acoustic delay of the cavitation signals. With reference to FIG. 8C, this is achieved by optimizing the propagation distance, D, of the acoustic signals through the skull 802 and housing 804 prior to reaching the cavitation detection device 806. For example, the location of the cavitation detection device 806 in the housing 804 may be adjusted such that D satisfies:

$$\mathrm{D} = d_1 + d_2 = n \times \lambda/2,$$

where $d_1$, $d_2$ represent the acoustic delay length of the skull 802 and the acoustic delay length of the housing portion 812, respectively, through which the cavitation signals travel prior to reaching the cavitation detection device 806; $\lambda$ represents the wavelength of the cavitation signals; and $n$ is an integer. In addition, the impedance of the housing 804, in particular the portion 812 through which the cavitation signals propagate prior to reaching the cavitation detection device 806, may be adjusted to provide impedance matching between the skull 802 and cavitation detection device 806, thereby maximizing the power received by the cavitation detection device 806. In one implementation, the impedance of the housing is controlled by adjusting the material properties of the housing. The housing portion 812 is designed to serve as an optimal acoustic transformer. For example, the material of the housing portion 812 may be chosen such that the acoustic properties thereof are substantially similar to that of the skull 802. In this way, the housing portion 812 and skull 802 behave as a continuous, single layer when the cavitation signals travel therethrough. Alternatively, the acoustic properties of the housing portion 812 may be different from that of the skull 802; thus, the acoustic velocity in the skull 802 may be different from that in the housing portion 812. To minimize the acoustic difference, the width, $D_h$, of the housing portion 812 is adjusted to satisfy:

$$\left(\frac{v_s}{D_s} + \frac{v_h}{D_h}\right) \times 2 = nT$$

where $D_s$ represents the width of the skull; $v_s$, $v_h$ represent the acoustic velocity in the skull 802 and in the housing portion 812, respectively; T represents the period of the acoustic waves; and $n$ is an integer.

Referring to FIG. 8D, the housing 804 includes an acoustic impedance-matching layer 814 (preferably in contact with the cavitation detection device 806) to further improve impedance matching between the skull 802 and cavitation detection device 806. Because different patients may have different skull impedances, varying the material properties of the housing and/or including the impedance-matching layer 814 may match the impedance of the cavitation detection device 806 (whose impedance generally is fixed) with that of the patient's skull (whose impedance is patient-specific).

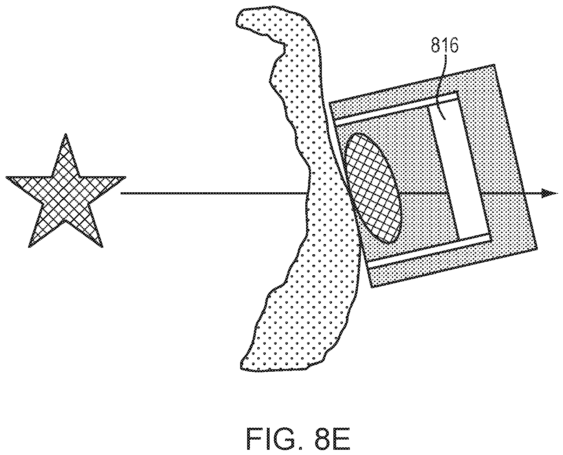

Referring to FIG. 8E, the housing 804 includes one or more acoustic absorbers and/or reflectors 816 to reduce the noise level in the received signals. The reflector may include any suitable material that has an acoustic impedance different from that of the surrounding material (i.e., the housing). For example, the reflector may be as simple as an air gap. Similarly, the absorber may include any suitable material that can effectively absorb the acoustic noise. In one implementation, the absorber is an off-the-shelf product (see, e.g., https://www.acoustics.co.uk/product-category/acoustic-materials/anechoic-absorbers/). The acoustic absorbers/reflectors 816 may effectively absorb/reflect signals originating from sources other than the target region 101 and/or signals that have been refracted, reflected and/or scattered and therefore cannot provide information accurately reflecting the cavitation effect on the target region 101.

Various approaches described herein for improving the SNR of the signals measured by the cavitation detection devices may be implemented alone or in combination with other approaches. For example, the cavitation detection devices may be attached to scalp regions corresponding to high transmission efficiency and the housing thereof may include both the impedance-matching layer 814 and acoustic absorbers/reflectors 816.

In general, functionality as described above (e.g., identifying locations of the scalp, skull and target region, analyzing images to acquire anatomical characteristics of the skull/scalp, predicting a beam path and beam aberrations travelling through each skull/scalp region, predicting transmission efficiency associated with each skull/scalp region, selecting the skull/scalp regions based on their transmission efficiencies, converting coordinates of an imaging system to the spatial coordinates of the environment, and/or mapping various skull regions to the scalp regions) whether integrated within a controller of the imaging system, a cavitation detection device 206 and/or an ultrasound system 100, or provided by a separate external controller or other computational entity or entities, may be structured in one or more modules implemented in hardware, software, or a combination of both. For implementations in which the functions are provided as one or more software programs, the programs may be written in any of a number of high level languages such as FORTRAN, PASCAL, JAVA, C, C++, C#, BASIC, various scripting languages, and/or HTML. Additionally, the software can be implemented in an assembly language directed to the microprocessor resident on a target computer (e.g., the controller); for example, the software may be implemented in Intel 80x86 assembly language if it is configured to run on an IBM PC or PC clone. The software may be embodied on an article of manufacture including, but not limited to, a floppy disk, a jump drive, a hard disk, an optical disk, a magnetic tape, a PROM, an EPROM, EEPROM, field-programmable gate array, or CD-ROM. Embodiments using hardware circuitry may be implemented using, for example, one or more FPGA, CPLD or ASIC processors.

In addition, the term "controller" used herein broadly includes all necessary hardware components and/or software modules utilized to perform any functionality as described above; the controller may include multiple hardware components and/or software modules and the functionality can be spread among different components and/or modules.

Certain embodiments of the present invention are described above. It is, however, expressly noted that the present invention is not limited to those embodiments; rather, additions and modifications to what is expressly described herein are also included within the scope of the invention as defined by the appended claims.

**Claims**

1. A system (100) for detecting cavitation signals from a target region (101) of a patient during a focused ultrasound procedure, the system comprising:

   an ultrasound transducer (102);
   an imaging device (112) for acquiring physiological characteristics of a plurality of anatomical regions through which the cavitation signals from the target region travel;
   a controller (108); and
   at least one cavitation detection device (206); **characterized in that**:

   the controller (108) is configured to:

   select at least one of the anatomical regions based at least in part on the physiological characteristics thereof; and
   map the selected anatomical region to a corresponding skin region; and

   the at least one cavitation detection device (206) is attachable to the corresponding skin region.

2. The system of claim 1, wherein the controller (108) is further configured to predict a beam path and beam aberrations of a cavitation signal travelling through each of the anatomical regions from the target region (101) based on the physiological characteristics of the anatomical regions along the beam path.

3. The system of claim 2, wherein the controller (108) is further configured to predict the transmission efficiency associated with each of the anatomical regions based on the physiological characteristics along the beam path.

4. The system of claim 3, wherein the physiological characteristics comprise at least one of a structure, a thickness, a number of layers, a local bone density, surface geometry, or an incidence angle of the beam path associated with each of the anatomical regions.

5. The system of claim 3, wherein the controller (108) is further configured to select at least one of the anatomical regions based on the transmission efficiency associated therewith.

6. The system of claim 1 or claim 2, wherein the controller (108) is further configured to map each said at least one anatomical region to a corresponding skin region by projecting a predicted signal path from the target region (101) onto the corresponding skin region.

7. The system of claim 1 or 6, the system further comprising a secondary imaging device for acquiring physiological characteristics of at least one of the target region (101) or corresponding skin region, wherein the controller (108) is further configured to register coordinates in the secondary imaging device to coordinates in the imaging device (112).

8. The system of claim 1, wherein the controller (108) is further configured to correlate coordinates of the imaging device (112) with spatial coordinates in a room in which the patient is located.

9. The system of claim 8 or claim 1, further comprising a secondary imaging device for acquiring a real-time image of at least three locational trackers (502).

10. The system of claim 9, wherein the controller (108) is further configured to register coordinates in the secondary imaging device to coordinates in the imaging device (112).

11. The system of claim 9, wherein the locational trackers (502) are attached to three fiducials, and at least one of the locational trackers (502) or the fiducials are detectable by the imaging device (112).

12. The system of claim 1, further comprising display hardware for displaying the corresponding skin region or generated map.

13. The system of claim 1, wherein the controller (108) is further configured to operate the ultrasound transducer (102) based at least in part on the cavitation signals received by the cavitation detection device (206).

14. A method of placing at least one cavitation detection device (206) for detecting cavitation signals from a target region (101) of a patient during a focused ultrasound procedure, the method comprising:

(a) acquiring characteristics of a plurality of anatomical regions through which the cavitation signals from the target region (101) travel;
(b) selecting at least one of the anatomical regions based at least in part on the characteristics thereof;
(c) mapping the selected anatomical region to a corresponding skin region; and
(d) based on the mapping, placing the at least one cavitation detection device (206) on the corresponding skin region.

**Patentansprüche**

1. System (100) zum Erkennen von Kavitationssignalen aus einer Zielregion (101) eines Patienten während einer Untersuchung mit fokussiertem Ultraschall, wobei das System umfasst:

ein Ultraschallwandler (102);
eine Bildgebungsvorrichtung (112) zum Erfassen physiologischer Eigenschaften einer Vielzahl von anatomischen Regionen, durch die die Kavitationssignale aus der Zielregion passieren;
eine Steuereinheit (108); und
mindestens eine Kavitationserkennungsvorrichtung (206);
**dadurch gekennzeichnet, dass**:

die Steuereinheit (108) konfiguriert ist zum:

Auswählen mindestens einer der anatomischen Regionen mindestens teilweise auf der Grundlage von deren physiologischen Eigenschaften; und
Abbilden der ausgewählten anatomischen Region auf einer entsprechenden Hautregion; und

wobei die mindestens eine Kavitationserkennungsvorrichtung (206) an der entsprechenden Hautregion anbringbar ist.

2. System nach Anspruch 1, wobei die Steuereinheit (108) ferner zum Vorhersagen eines Strahlengangs und von Strahlaberrationen eines Kavitationssignals, das aus der Zielregion (101) durch jede der anatomischen Regionen passiert, auf der Grundlage der physiologischen Eigenschaften der anatomischen Regionen entlang des Strahlengangs konfiguriert ist.

3. System nach Anspruch 2, wobei die Steuereinheit (108) ferner zum Vorhersagen des jeder der anatomischen Regionen zugeordneten Übertragungswirkungsgrads auf der Grundlage der physiologischen Eigenschaften entlang des Strahlengangs konfiguriert ist.

4. System nach Anspruch 3, wobei die physiologischen Eigenschaften mindestens eine von einer Struktur, einer Dicke, einer Anzahl von Schichten, einer lokalen Knochendichte, einer Oberflächengeometrie oder eines Einfallswinkels des Strahlengangs umfassen, der jeder der anatomischen Regionen zugeordnet ist.

5. System nach Anspruch 3, wobei die Steuereinheit (108) ferner zum Auswählen mindestens einer der anatomischen Regionen auf der Grundlage des ihnen zugeordneten Übertragungswirkungsgrades konfiguriert ist.

6. System nach Anspruch 1 oder Anspruch 2, wobei die Steuereinheit (108) ferner zum Abbilden jeder der mindestens einen anatomischen Region auf einer entsprechenden Hautregion konfiguriert ist, indem ein vorhergesagter Signalweg von der Zielregion (101) auf die entsprechende Hautregion projiziert wird.

7. System nach Anspruch 1 oder 6, wobei das System ferner eine sekundäre Bildgebungsvorrichtung zum Erfassen physiologischer Eigenschaften mindestens einer von der Zielregion (101) oder der entsprechenden Hautregion umfasst, wobei die Steuereinheit (108) ferner zum Registrieren von Koordinaten in der sekundären Bildgebungsvorrichtung auf Koordinaten in der Bildgebungsvorrichtung (112) konfiguriert ist.

8. System nach Anspruch 1, wobei die Steuereinheit (108) ferner zum Korrelieren von Koordinaten der Bildgebungsvorrichtung (112) mit Raumkoordinaten in einem Raum konfiguriert ist, in dem sich der Patient befindet.

9. System nach Anspruch 8 oder Anspruch 1, ferner umfassend eine sekundäre Bildgebungsvorrichtung zum Erfassen eines Echtzeitbildes von mindestens drei Lageverfolgungseinheiten (502).

10. System nach Anspruch 9, wobei die Steuereinheit (108) ferner zum Registrieren von Koordinaten in der sekundären Bildgebungsvorrichtung auf Koordinaten in der Bildgebungsvorrichtung (112) konfiguriert ist.

11. System nach Anspruch 9, wobei die Lageverfolgungseinheiten (502) an drei Referenzpositionen angebracht sind und mindestens eine der Lagerverfolgungseinheiten (502) oder die Referenzpositionen durch die Bildgebungsvorrichtung (112) erkennbar sind.

12. System nach Anspruch 1, ferner umfassend Anzeigehardware zum Anzeigen der entsprechenden Hautregion oder der erzeugten Abbildung.

13. System nach Anspruch 1, wobei die Steuereinheit (108) ferner zum Betreiben des Ultraschallwandlers (102) mindestens teilweise auf der Grundlage der von der Kavitationserkennungsvorrichtung (206) empfangenen Kavitationssignale konfiguriert ist.

14. Verfahren zum Platzieren mindestens einer Kavitationserkennungsvorrichtung (206) zum Erkennen von Kavitationssignalen aus einer Zielregion (101) eines Patienten während einer Untersuchung mit fokussiertem Ultraschall, wobei das Verfahren umfasst:

(a) Erfassen von Eigenschaften einer Vielzahl von anatomischen Regionen, durch die die Kavitationssignale aus der Zielregion (101) passieren;
(b) Auswählen mindestens einer der anatomischen Regionen mindestens teilweise auf der Grundlage von deren Eigenschaften;
(c) Abbilden der ausgewählten anatomischen Region auf einer entsprechenden Hautregion; und
(d) auf der Grundlage der Abbildung Platzieren der mindestens einen Kavitationserkennungsvorrichtung (206) auf der entsprechenden Hautregion.

**Revendications**

1. Système (100) destiné à une détection de signaux de cavitation provenant d'une région cible (101) d'un patient pendant une procédure à ultrasons focalisés, le système comprenant :

un transducteur à ultrasons (102) ;
un dispositif d'imagerie (112) servant à acquérir des caractéristiques physiologiques d'une pluralité de régions anatomiques à travers lesquelles se propagent les signaux de cavitation provenant de la région cible ;
un dispositif de commande (108) ; et
au moins un dispositif de détection de cavitation (206) ;
**caractérisé en ce que** :

le dispositif de commande (108) est configuré pour :

sélectionner au moins une des régions anatomiques sur la base, au moins en partie, de ses caractéristiques physiologiques ; et
mapper la région anatomique sélectionnée sur une région cutanée correspondante ; et

l'au moins un dispositif de détection de cavitation (206) peut être fixé à la région cutanée correspondante.

2. Système selon la revendication 1, dans lequel le dispositif de commande (108) est en outre configuré pour prédire un trajet de faisceau et des aberrations de faisceau d'un signal de cavitation traversant chacune des régions anatomiques à partir de la région cible (101) sur la base des caractéristiques physiologiques des régions anatomiques le long du trajet de faisceau.

3. Système selon la revendication 2, dans lequel le dispositif de commande (108) est en outre configuré pour prédire l'efficacité de transmission associée à chacune des régions anatomiques sur la base des caractéristiques physiologiques le long du trajet de faisceau.

4. Système selon la revendication 3, dans lequel les caractéristiques physiologiques comprennent au moins un élément parmi une structure, une épaisseur, un nombre de couches, une densité osseuse locale, une géométrie de surface ou un angle d'incidence du trajet de faisceau associé à chacune des régions anatomiques.

5. Système selon la revendication 3, dans lequel le dispositif de commande (108) est en outre configuré pour sélectionner au moins une des régions anatomiques sur la base de l'efficacité de transmission qui lui est associée.

6. Système selon la revendication 1 ou la revendication 2, dans lequel le dispositif de commande (108) est en outre configuré pour mapper chacune desdites au moins une région anatomique sur une région cutanée correspondante en projetant un trajet de signal prédit depuis la région cible (101) sur la région cutanée correspondante.

7. Système selon la revendication 1 ou 6, le système comprenant en outre un dispositif d'imagerie secondaire servant à acquérir des caractéristiques physiologiques d'au moins une région parmi la région cible (101) ou une région cutanée correspondante, dans lequel le dispositif de commande (108) est en outre configuré pour enregistrer des coordonnées dans le dispositif d'imagerie secondaire dans des coordonnées dans le dispositif d'imagerie (112).

8. Système selon la revendication 1, dans lequel le dispositif de commande (108) est en outre configuré pour corréler des coordonnées du dispositif d'imagerie (112) avec des coordonnées spatiales dans une pièce dans laquelle se trouve le patient.

9. Système selon la revendication 8 ou la revendication 1, comprenant en outre un dispositif d'imagerie secondaire servant à acquérir une image en temps réel d'au moins trois dispositifs de suivi de localisation (502).

10. Système selon la revendication 9, dans lequel le dispositif de commande (108) est en outre configuré pour enregistrer des coordonnées dans le dispositif d'imagerie secondaire dans des coordonnées dans le dispositif d'imagerie (112).

11. Système selon la revendication 9, dans lequel les dispositifs de suivi de localisation (502) sont fixés à trois repères, et au moins l'un des dispositifs de suivi de localisation (502) ou des repères sont détectables par le dispositif d'imagerie (112).

12. Système selon la revendication 1, comprenant en outre un matériel d'affichage pour afficher la région cutanée ou la carte générée correspondante.

13. Système selon la revendication 1, dans lequel le dispositif de commande (108) est en outre configuré pour faire fonctionner le transducteur à ultrasons (102) sur la base, au moins en partie, des signaux de cavitation reçus par le dispositif de détection de cavitation (206).

14. Procédé de mise en place d'au moins un dispositif de détection de cavitation (206) permettant une détection de signaux de cavitation provenant d'une région cible (101) d'un patient pendant une procédure à ultrasons focalisés, le procédé comprenant :

(a) l'acquisition de caractéristiques d'une pluralité de régions anatomiques à travers lesquelles se propagent les signaux de cavitation provenant de la région cible (101) ;
(b) la sélection d'au moins une des régions anatomiques sur la base, au moins en partie, de ses caractéristiques ;
(c) le mappage de la région anatomique sélectionnée sur une région cutanée correspondante ; et
(d) sur la base du mappage, la mise en place de l'au moins un dispositif de détection de cavitation (206) sur la région cutanée correspondante.

FIG. 1

EP 3 684 263 B1

FIG. 2A

FIG. 2B

FIG. 3A

FIG. 3B

FIG. 4A

FIG. 4B

FIG. 5

FIG. 6

700

ACQUIRE A SERIES OF IMAGES OF A PATIENT'S HEAD ～702

PROCESS THE IMAGES TO ACQUIRE INFORMATION OF THE TARGET REGION, THE SCALP AND THE SKULL ～704

DETERMINE ANATOMICAL CHARACTERISTICS ASSOCIATED WITH EACH SKULL AND/OR SCALP REGION ～706

PREDICT A BEAM PATH AND BEAM ABERRATIONS TRAVELLING THROUGH EACH SKULL/SCALP REGION FROM THE TARGET REGION ～708

DETERMINE TRANSMISSION EFFICIENCY ASSOCIATED WITH EACH SKULL/SCALP REGION ～710

SELECT SKULL/SCALP REGIONS WHERE THE CAVITATION DETECTION DEVICES ARE TO BE ATTACHED (OPTIONAL) ～712

CONVERT COORDINATES OF THE IMAGING SYSTEM TO THE SPATIAL COORDINATES OF THE ENVIRONMENT ～714

DISPLAY THE SKULL/SCALP REGIONS AND LOCATIONS OF THE MR TRACKERS ～716

ATTACH THE CAVITATION DETECTION DEVICES ONTO THE SKULL REGIONS ～718

FIG. 7

FIG. 8A

FIG. 8B

FIG. 8C

FIG. 8D

FIG. 8E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017004562 A
- US 415351
- US 20170103533 A
- US 20160184026 A